Europäisches Patentamt

European Patent Office

Office européen des brevets

(19)

(11) Veröffentlichungsnummer: **0 031 588**
**B1**

(12) EUROPÄISCHE PATENTSCHRIFT

(45) Veröffentlichungstag der Patentschrift:
**29.12.82**

(21) Anmeldenummer: **80108158.9**

(22) Anmeldetag: **23.12.80**

(51) Int. Cl.³: **C 07 C 11/24**, C 07 C 1/00,
B 01 J 19/00

(54) **Verfahren zur Herstellung von Acetylen aus Kohle.**

(30) Priorität: **28.12.79 DE 2952519**

(43) Veröffentlichungstag der Anmeldung:
**08.07.81 Patentblatt 81/27**

(45) Bekanntmachung des Hinweises auf die Patenterteilung:
**29.12.82 Patentblatt 82/52**

(84) Benannte Vertragsstaaten:
**BE CH DE FR GB LI LU NL SE**

(56) Entgegenhaltungen:
**DD-A-114 395**
**GB-A-1 089 092**
**NATURE, Band 236, Nr. 5347, 21. April 1972, Seiten 397—400 London, G. B. R. NICHOLSON et al.: »Plasma pyrolysis of coal«**

(73) Patentinhaber: **Bergwerksverband GmbH,
Franz-Fischer-Weg 61, D-4300 Essen 13 (DE)**

(72) Erfinder: **Peuckert, Cornelius, Dipl.-Chem.,
Weststrasse 35, D-4220 Dinslaken (DE)**
Erfinder: **Baumann, Herbert, Dipl.-Phys. Dr., Frillendorfer Höhe 24, D-4300 Essen (DE)**
Erfinder: **Klein, Jürgen, Dipl.-Phys. Dr., Hülscherfeld 32, D-4300 Essen 14 (DE)**
Erfinder: **Bittner, Dirk, Dipl.-Chem., Emmastrasse 70, D-4300 Essen (DE)**
Erfinder: **Jüntgen, Harald, Dipl.-Chem. Prof. Dr., Bonscheidter Strasse 79, D-4300 Essen 15 (DE)**

(74) Vertreter: **Schumacher, Horst, Dr., Bergwerksverband GmbH Patent- und Vertragsabteilung Franz-Fischer-Weg 61, D-4300 Essen 13 (DE)**

0 031 588

## Verfahren zur Herstellung von Acetylen aus Kohle

Die Erfindung betrifft ein Verfahren gemäß dem Oberbegriff von Patentanspruch 1 sowie eine Vorrichtung zum Durchführen dieses Verfahrens.

Acetylen entsteht aus anderen Kohlenwasserstoffen bei Temperaturen über ca. 1400°C. Da es instabil ist, würde es ohne Gegenmaßnahmen nach kurzer Zeit bis zur Gleichgewichtskonzentration, die bei 1400°C sehr niedrig ist und bei tieferen Temperaturen noch niedriger wird, zerfallen. Deshalb muß man die Temperatur in dem Augenblick, in dem die Acetylenkonzentration am höchsten ist, plötzlich erniedrigen (quenchen), um diesen Zustand hoher Acetylenkonzentration einzufrieren. Diese plötzliche Temperaturerniedrigung kann durch Eindüsen von Wasser, von gasförmigen, flüssigen oder festen Kohlenwasserstoffen, durch rezirkulierte Produkte oder auf andere Weise erfolgen.

Zur Erzeugung der für die Acetylenbildung notwendigen hohen Temperaturen kann man alle an sich bekannten Methoden verwenden, die die notwendig schnelle Aufheizung der Kohlepartikel von mindestens etwa $10^5$ °C/s gewährleisten können. Zum Beispiel ist es bekannt, hierfür eine Lichtbogenentladung zu benutzen. — Bei letzterem sind Gleich- und Wechselstrom (Drehstrom) in gleicher Weise geeignet. Das Verhältnis von Stromstärke zu Spannung hängt von der Bogenlänge und vom Bogengas ab und ist durch die Reaktorgeometrie und die Fahrweise — z. B. mit oder ohne Gaszirkulierung — festgelegt. Es eignen sich dabei sowohl Verfahrensweise mit einem Strom/Spannungsverhältnis größer als eins als auch solche mit einem Strom/Spannungsverhältnis kleiner als eins. Zur Stabilisierung des Lichtbogens kann man beispielsweise einen Gaswirbel verwenden (Proc. 8th World Petroleum Congress (1971), Band 4, Seiten 379–388) oder ein Magnetfeld, das den Lichtbogen in Rotation versetzt (US-PS 3 073 769).

Es ist bekannt, Acetylen aus gasförmigen oder flüssigen Kohlenwasserstoffen, beispielsweise von Methan bis Rohöl, herzustellen (Ullmanns Enzyklopädie der technischen Chemie, 4. Auflage (1974), Band 7, Seiten 47–49).

Es ist weiter bekannt, auch aus bituminösen Kohlen mit einem Gehalt an flüchtigen Bestandteilen über etwa 38 Gew.-% (auf wasser- und aschefreier Basis = i. waf) im Lichtbogenreaktor Acetylen herzustellen, wobei jedoch die Acetylenausbeute nur bei 7–9 Gew.-% lag (P. H. Given, Report to Office of Coal Research, U. S. State Department of Interior, Report No. 61, Interim Report No. 5, Seiten 4 und 5).

Schließlich ist aus der Zeitschrift Nature 236 (Apr. 21, 1972), Seiten 397–400, ein Verfahren der eingangs genannten Art bekannt, bei welchem bei der Umsetzung von Kohle im Lichtbogen Acetylenausbeuten bis zu 74%, bezogen auf den eingesetzten Kohlenstoff, erreicht werden; hierzu mußten aber mehr als 8000 kWh elektrische Energie pro kg Acetylen aufgebracht werden.

Die Wirtschaftlichkeit eines Verfahrens zur Herstellung von Acetylen aus Kohle hängt wesentlich von der Acetylenausbeute, der Acetylenkonzentration im Produktgas und dem spezifischen Energiebedarf (Energiebedarf zur Erzeugung von 1 kg Acetylen) ab: die Acetylenausbeute und -konzentration müssen möglichst hoch und der spezifische Energiebedarf möglichst niedrig sein.

Der Erfindung liegt demnach im wesentlichen die Aufgabe zugrunde, bei einem Verfahren der gattungsgemäßen Art aus Kohle Acetylen in möglichst hoher Ausbeute bei einem möglichst niedrigen spezifischen Energiebedarf herzustellen, sowie eine Vorrichtung zum Durchführen dieses Verfahrens zu schaffen.

Diese Aufgabe wird bei einem Verfahren der gattungsgemäßen Art erfindungsgemäß durch die kennzeichnenden Merkmale des Anspruches 1 gelöst.

Der Erfindung liegt die Erkenntnis zugrunde, daß die Bildung des Acetylens mit seinem Zerfall konkurriert, so daß im zeitlichen Reaktionsablauf ein Maximum der Konzentrationen des Acetylens und der Radikale, die beim Abschrecken Acetylen bilden, entsteht. Für eine optimale Acetylenerzeugung ist es also erfindungsgemäß erforderlich, daß an der Stelle im Reaktor, an der dieses Maximum erreicht wird, die Produkte abgeschreckt (gequencht) werden.

Die Acetylenausbeute wird höher, wenn die Pyrolyse der Kohle schneller abläuft, denn bei einer schnellen Pyrolyse ist am Ende der Pyrolyse weniger von dem anfangs gebildeten Acetylen zerfallen als bei einer langsamen.

Es war zwar bisher bekannt, daß eine feiner gemahlene Kohle, die dementsprechend eine sehr große spezifische äußere Oberfläche aufweist, höhere Acetylenausbeuten ermöglicht als nicht so fein aufgemahlene Kohle. Es mußte jedoch durchaus überraschen, daß eine feingemahlene Kohle bei der Verarbeitung zu Acetylen ohne vorheriges Fraktionieren in verschiedene Kornfraktionen eine geringere Acetylenausbeute ergibt als dann, wenn man diese Kohle in eine gröbere und eine feinere Kornfraktion trennt und dann nur die gröbere Kornfraktion für die Acetylenerzeugung — unter Berücksichtigung der erfindungsgemäßen Maßnahmen — verwendet. Die Berücksichtigung der erfindungsgemäßen Maßnahmen bewirkt bei der feineren Kornfraktion im übrigen eine über das für die Feinheit der Mahlung bekannte Maß hinausgehende Steigerung der Acetylenausbeute.

Die Frage nach der Zahl der voneinander zu trennenden Kornfraktionen wird durch die Wirtschaftlichkeit des Verfahrens bestimmt; bereits eine Trennung in zwei Kornfraktionen erbringt eine erhebliche Steigerung der Acetylenausbeute und diese wird durch noch feinere Fraktionierung

2

verbessert. Wahrscheinlich wird aber bei mehr als vier Kornfraktionen der dafür notwendige Trennvorgang unverhältnismäßig teuer und deshalb uninteressant sein.

Die einzelnen Kornfraktionen können sowohl nach einer Zwischenspeicherung in größeren Zeitabständen nacheinander in ein und demselben Reaktor verarbeitet werden oder aber in zwei parallel arbeitenden, im wesentlichen aber gleich gestalteten Reaktoren eingesetzt werden.

Das beste Ergebnis wird erzielt, wenn bei konstanten Reaktionsbedingungen zunächst für die Gesamtfraktion aller Kohlepartikel, sowie sie nach der Mahlung vorliegt, die beste Verweilzeit gemäß Anspruch 1 ermittelt wird und dann für die einzelnen Kornfraktionen die Verweilzeiten so eingestellt werden, daß die sich zu der als optimal für die Gesamtfraktion ermittelten Verweilzeit verhalten, wie das umgekehrte Verhältnis der dritten Wurzel der spezifischen äußeren Oberflächen der jeweiligen Kornfraktion zur Gesamtfraktion.

Sofern die äußeren Bedingungen der Gesamtanlage, insbesondere der Reaktionsstrecke, es nicht zulassen, die Verweilzeiten in dem durch die erfindungsgemäße Vorschrift vorgeschlagenem Maße zu ändern, so werden auch noch gute Ergebnisse erzielt, wenn anstelle der dritten Wurzeln die 2,5ten bis 3,5ten Wurzeln der spezifischen äußeren Oberflächen der einzelnen Kornfraktionen zur Grundlage genommen werden.

Die genannten dritten Wurzeln gelten für ansonsten gleiche Reaktionsbedingungen und treffen nicht mehr ganz genau zu, wenn verfahrenswesentliche Parameter für unterschiedliche Kornfraktionen verschieden gewählt werden; in diesem Falle soll bei der Festlegung der Verweilzeit berücksichtigt werden, in welcher Weise die für die einzelnen Fraktionen unterschiedlichen Parameter — mit Ausnahme der Verweilzeit — voraussichtlich auch auf die Verweilzeiten Einfluß nehmen.

Die erfindungsgemäß vorgeschlagene Variation der Verweilzeit der Kohle in der Reaktionsstrecke kann erfindungsgemäß entweder dadurch erreicht werden, daß man die Länge der Reaktionsstrecke oder die Strömungsgeschwindigkeit der Kohle in derselben variiert. Dabei empfiehlt es sich für die erstgenannte Lösung, das Abschrecken an verschiedenen Stellen der Reaktionsstrecke vornehmen zu können — vorzugsweise an jeder beliebigen. Bei der zweiten Lösung bleibt die Länge der Reaktionsstrecke konstant, während die Strömungsgeschwindigkeit der Kohle z. B. durch einen entsprechend schnell eingestellten, die Kohle aufheizenden Gasstrom variiert werden kann, wie durch Querschnittsverengung in der Reaktionsstrecke. — Die übrigen Verfahrensparameter bleiben ansonsten jeweils konstant und richten sich nach den Auslegungsdaten der für einen bestimmten Kohledurchsatz bestimmten Anlage zur Acetylenherstellung.

Es hat sich als besonders günstig herausgestellt, wenn erfindungsgemäß die spezifische äußere Oberfläche der Kohle (nach der Blaine-Methode bestimmt) zwischen 0,5 und 2,0 $m^2/cm^3$ beträgt. In diesem Bereich ist sowohl die Mahlung als auch die Trennung der gemahlenen Kohle in einzelne Kornfraktionen mit wirtschaftlich vertretbarem Aufwand gut möglich und es ergeben sich dabei sehr zufriedenstellende Acetylenausbeuten bei vergleichsweise geringem Energieeinsatz.

Eine weitere Verbesserung läßt sich erfindungsgemäß dadurch erzielen, daß die Trennung der feingemahlenen Kohle in einzelne Kornfraktionen so vorgenommen wird, daß bei der Korngrößenverteilung jeder Fraktion die mittlere Steigung der Körnungskennlinie im Rosin-Rammler-Sperling-Bennet-Netz (RRSB-Verteilung) zwischen 1,2 und 2,5 liegt. Die letztgenannte Größe wird üblicherweise als Gleichmäßigkeitsparameter (n) bezeichnet und ist unter anderem aus Ullmanns Enzyklopädie der technischen Chemie, 4. Auflage, Band 2, Seite 27, bekannt. Größere Steigerungen sind dabei günstiger, jedoch nur durch einen besonders hohen Aufwand bei der Trennung erzielbar. Demgegenüber führen besonders kleine Gleichmäßigkeitsparameter zu weniger guten Ausbeuteergebnissen, wenn man z. B. zwei Fraktionen mit gleicher spezifischer äußerer Oberfläche der Kohlepartikel vergleicht.

Als Ausgangsmaterial für die Herstellung von Acetylen eignen sich grundsätzlich alle Kohlen von Braunkohlen über die verschieden hoch entkohlten Steinkohlen bis zu Anthraziten. Die Acetylenausbeute ist aber vom Inkohlungsgrad der Kohle bekanntlich abhängig. Ebenso spielt der Gehalt der Kohle an organischem Sauerstoff eine wichtige Rolle für die mögliche Acetylenausbeute. Der Sauerstoff aus der Kohle verbindet sich nämlich beim schnellen Erhitzen mit einem Teil des flüchtigen Kohlenstoffes zu Kohlenmonoxid, so daß dieser Kohlenstoff für die Acetylenbildung nicht zur Verfügung stehen.

Beispielsweise ist die Acetylenausbeute bei Braunkohlen, die einen höheren Anteil an organisch gebundenem Sauerstoff besitzen als z. B. höher inkohlte Kohlen geringer als bei hochflüchtigen Steinkohlen, obwohl Braunkohlen einen höheren Gehalt an flüchtigen Bestandteilen besitzen. Entsprechend wird erfindungsgemäß vorgeschlagen, daß die zu Acetylen zu verarbeitenden Kohlen einen Gehalt von flüchtigen Bestandteilen (i. waf) zwischen 25 und 44%, vorzugsweise zwischen 30 und 40% und einen Gehalt an organischem Sauerstoff (i. waf) unter 9%, vorzugsweise unter 7% aufweisen sollen.

Es hat sich als besonders vorteilhaft erwiesen, wenn gemäß einer Weiterbildung der Erfindung die Aufheizung der Kohle in einem Wasserstoff enthaltenden Gasstrom erfolgt, da hierdurch sowohl die Acetylenbildung unterstützt als auch sehr viel fühlbare Wärme bei nicht allzu hohen Temperaturen auf die Kohle übertragen werden kann. Es hat sich nämlich herausgestellt, daß für die Acetylenbildung Temperaturen zwischen 2000 und 3000°C besonders günstig sind, während bei niedrigeren

3

Temperaturen die Acetylenbildung sehr träge ist und bei höheren Temperaturen unnötig viel Energie eingesetzt werden muß. Der besagte Gasstrom sollte möglichst wenig Komponente enthalten, die das produzierte Acetylen unnötig verdünnen, bzw. seine Bildung beeinträchtigen oder vermindern wie dies z. B. bei Sauerstoff der Fall ist. Für den die Kohle aufheizenden Gasstrom kommen neben Wasserstoff unter anderem inerte Gase wie Edelgase in Frage.

Dabei hat es sich als besonders günstig erwiesen, wenn erfindungsgemäß das Wasserstoff enthaltende Gas durch einen Lichtbogen mit einer Energie von 100 bis 500 kJ/mol aufgeheizt wird, da hierdurch eine sehr hohe Energiedichte und nicht unnötig hohe Temperaturen im Aufheizgas bereitgestellt werden können.

Es ist aber erfindungsgemäß auch möglich, die Energie zum Aufheizen der Kohle durch Verbrennung eines kohlenstoffhaltigen Materials unter weitgehender Vermeidung von $CO_2$ und $O_2$ im Rauchgas bereitzustellen. Bei dem kohlenstoffhaltigen Material kann es sich um gasförmige, flüssige oder feste Produkte handeln, die in möglichst reinem Sauerstoff verbrannt werden.

Unabhängig von der Energiequelle zum Aufheizen der Kohle hat es sich als besonders vorteilhaft erwiesen, wenn erfindungsgemäß das Verhältnis von der thermisch verwertbaren Leistung der Energiequelle zur Strömung an Kohlepartikeln 4000 bis 40 000 kJ/kg Kohle beträgt. Es hat sich nämlich gezeigt, daß in diesem Bereich die Acetylenausbeute ihre besten Werte bei gleichzeitiger Einschränkung der hierfür notwendigen Energie auf ein Minimum möglich ist.

Gemäß einer Weiterbildung der Erfindung empfiehlt sich die Rückführung von nach der Reaktionsstrecke anfallenden kohlenstoffhaltigen Rückständen, sofern die Aufheizung der feingemahlenen Kohle durch Verbrennung eines kohlenstoffhaltigen Materials erfolgt. Hierdurch wird unter anderem das Problem gelöst, daß der koksähnliche Rückstand der in der Regel aus der zur Acetylenherstellung eingesetzten feingemahlenen Kohle, der noch verwertbares kohlenstoffhaltiges Material beinhaltet, weiter verwertet werden kann und ggfs. sogar schon allein ausreicht, um bei seiner Verbrennung die für das Aufheizen notwendige Energie zu liefern.

Die Aufgabe der Erfindung wird in bezug auf eine Vorrichtung zum Durchführen des Verfahrens gemäß dem Hauptanspruch durch einen länglichen Reaktionsraum für die Durchströmung mit Kohle gelöst, bei dem je eine Einspeisestelle für Kohle und Aufheizenergie an dem einen Endbereich vorgesehen ist, sowie eine längs des Reaktionsraumes variierbare Einspeisestelle für das Abschreckmedium und ein Austritt für Produktgas und Rückstandsmaterial an dem anderen Endbereich. Vorzugsweise handelt es sich bei der variierbaren Einspeisestelle für das Abschreckmedium um eine axial im Reaktor verschiebbare Aufgabelanze mit einer Austrittsöffnung für das Abschreckmedium — und schließlich ist es auch möglich, die Einspeisung für das Abschreckmedium dadurch längs des Reaktionsraumes zu variieren, daß an seiner Peripherie angeordnete Düsen vorgesehen sind; die jeweils zu einer Querschnittsebene des Reaktionsraumes gehörenden Düsen sollen dabei gemeinsam mit dem Abschreckmedium beaufschlagt werden, während alle übrigen Düsen in den anderen Querschnittsebenen des Reaktionsraumes von der Zufuhr für das Abschreckmedium abgesperrt sind. Es ist von besonderem Vorteil, wenn die Austrittsöffnungen für das Abschreckmedium so angeordnet sind, daß sich eine definierte Querschnittsebene im Reaktionsraum ausbildet, bei der die Abschreckung der zuvor aufgeheizten feingemahlenen Kohlepartikel und des Gases beginnt.

Weitere Ziele, Vorteile, Merkmale und Anwendungsmöglichkeiten der vorliegenden Erfindung ergeben sich aus der nachfolgenden Beschreibung eines Ausführungsbeispieles und anhand der beiliegenden Zeichnung. Dabei bilden alle beschrieben und/oder bildlich dargestellten Merkmale für sich oder in beliebiger sinnvoller Kombination den Gegenstand der vorliegenden Erfindung.

In den Figuren zeigt

Fig. 1 die Abhängigkeit der Korngrößen verschiedener Kornfraktionen in Mikrometern von ihrer Häufigkeit;

Fig. 2 einen Reaktionsraum für die Durchführung des erfindungsgemäßen Verfahrens, zum Teil schematisch und im Schnitt.

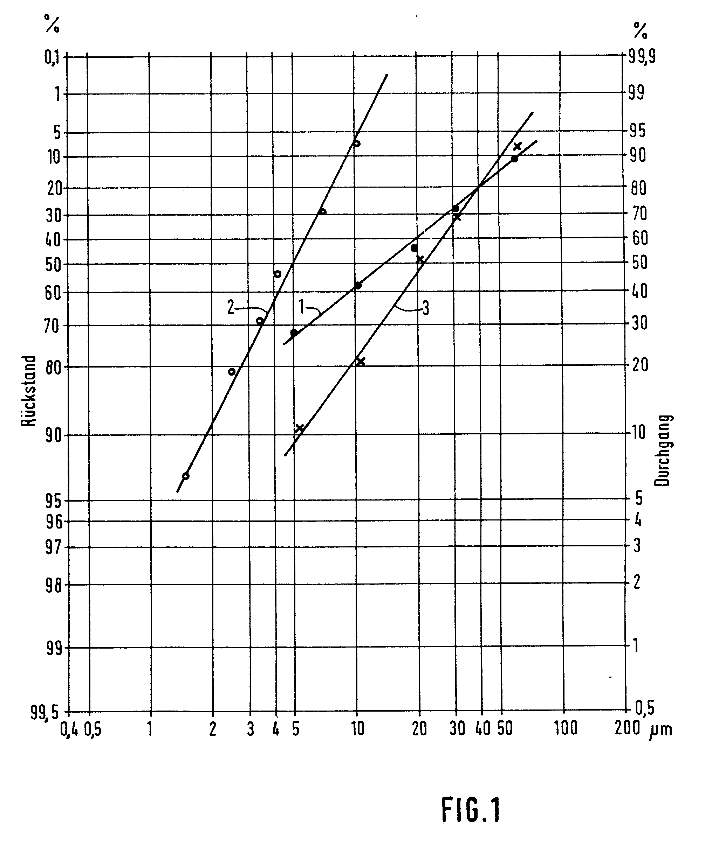

In Fig. 1 wird durch 1 eine Korngrößenverteilung charakterisiert, bei der sowohl kleinere als auch größere Korndurchmesser mit Häufigkeiten von nicht unerheblichem Umfange vorliegen. Es handelt sich also um eine »breite« Korngrößenverteilung und der Gleichmäßigkeitsparameter beträgt etwa 0,8. Mit 2 und 3 werden zwei Kornfraktionen dargestellt, die durch Trennung der Kornfraktion 1 entstanden sind, wobei es sich bei 2 um das Feinkorn und bei 3 um das Grobkorn handelt. Die Gleichmäßigkeitsparameter liegen beim Feinkorn bei 2,0 und beim Grobkorn bei 1,4.

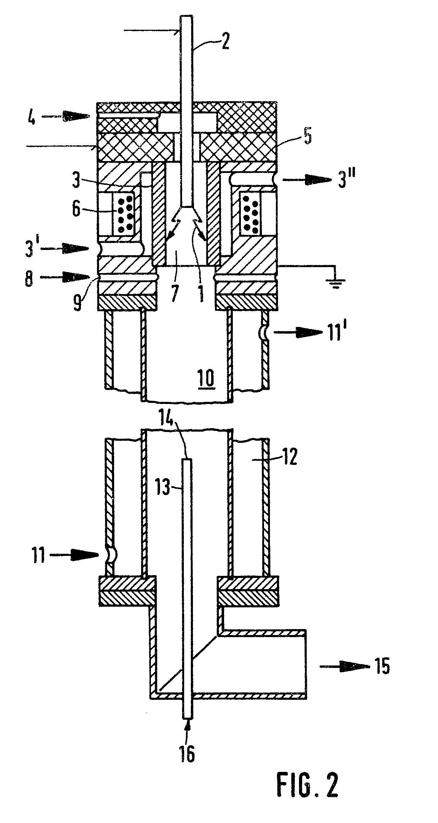

In Fig. 2 brennt der Gleichstrom-Lichtbogen 1 zwischen der stabförmigen Kathode 2 aus Wolfram-Thorium und der wassergekühlten 3', 3'', hohlzylinderförmigen Anode 3 aus Kupfer in einer Wasserstoff enthaltenden Gasströmung 4. Ein Isolator 5 sorgt für die galvanische Trennung von Kathode und Anode. Ein Magnetfeld, das durch die mit Gleichstrom versorgte Spule 6 im Innern der Anode erzeugt wird, versetzt den Lichtbogen 1 in Rotation. Nach Austritt aus dem Anodenraum 7 (Einspeisestelle für Aufheizenergie) wird dem aufgeheizten Gas Kohle 8 zugemischt, die mit Hilfe von kaltem Gas durch Düsen 9 (Eispeisestelle für Kohle) eingeführt wird. Die Kohle strömt zusammen mit dem Gas durch den Reaktionsraum 10, der als von außen mit Wasser 11, 11' gekühlter Hohlzylinder 12 ausgebildet ist. In dem Reaktionsraum ragt eine axial verschiebbare hohle Aufgabelanze 13

4

(Einspeisestelle für Abschreckmedium), aus deren Spitze 14 das zum Abschrecken der Reaktionsprodukte 15 (Austritt) dienende kalte Medium 16 in den Reaktionsraum 10 eintritt.

Ausführungsbeispiel

Für alle Ausführungsbeispiele wurde zur Aufheizung der umzuwandelnden Kohle ein Lichtbogenreaktor verwendet, der mit Gleichstrom von 200 Ampere betrieben wurde. Die Kathode bestand aus einer 4 mm starken Wolfram-Thorium-Nadel, die in der Mitte einer 5 cm langen Kupferanode, die die Form eines Hohlzylinders mit einem Innendurchmesser von 1 cm hatte, zentriert war. Mit einem Argondurchsatz von 0,6 mol/min durch die Anode und mit einer Spannung von etwa 2000 V wurde der Lichtbogen gezündet. Eine außen um die Anode gelegte Magnetspule, die in der Anodenachse ein Magnetfeld von 10 000 A/m erzeugte, versetzte den Lichtbogen in Rotation. Bei einer Wasserstoffzugabe von 4 mol/min durch die Anode stellte sich eine Betriebsspannung von 130 V ein. Die Anode wurde mit einem Kühlwasserdurchsatz von 4 l/min von außen gekühlt. Aus der Temperaturerhöhung des Kühlwassers errechnete sich eine (Verlust)-Leistung im Kühlwasser von 6,3 kW, so daß 19,7 kW zur Erhöhung der Kathodengasenthalpie zur Verfügung stand. In das so aufgeheizte Gas wurde mit 2 mol/min Wasserstoff 84 g/min Kohle unmittelbar unterhalb der Anode in ein mit Leitungswasser gekühltes vertikales Reaktionsrohr aus Kupfer mit einem Innendurchmesser von 2,4 cm radial eingeblasen. Der sich im Reaktionsraum ausbildende Druck lag bei etwa 1 bar. Die eingesetzte Kohle war eine Steinkohle mit einer Körnung 0,5 mm und einem Gehalt an flüchtigen Bestandteilen (i. waf) von 38,6 Gew.-% und einem Gehalt an organischen Sauerstoff (i. waf) von 8,2 Gew.-%. Die Eindüsung der Kohle erfolgte in einem ziemlich genau definierten Querschnitt des Reaktionsraumes. — Der insgesamt in den Reaktionsraum eingeführte Gasstrom betrug etwa 7 mol/min und bestand zu 91 Vol.-% aus Wasserstoff und zu 9 Vol.-% aus Argon. ·

Im Reaktionsrohr wurde die Kohle im weiter unten aufgeführten Umfange zu Acetylen umgesetzt. Die dabei entstehenden Reaktionsprodukte wurden jeweils in einem definierten Querschnitt des Reaktionsrohres mit einer Menge von 8 mol/min bei einem Druck von 1 bar und 25°C, in der Art abgekühlt, daß der Wasserstoff durch eine axial ausgerichtete und innerhalb des Reaktionsrohres verschiebbare Lanze in diese eingeblasen wurde, so daß die Temperatur nach diesem Abschrecken (Quenchen) kleiner als 1000°C war. — Mehr Kühlmittel hätte zwar die Endtemperatur erniedrigt, aber gleichzeitig auch durch Verdünnung die Acetylenkonzentration vermindert.

Versuchsreihe 1

Die oben beschriebene Kohle wurde so gemahlen, daß ihre Blaine-Oberfläche 1,26 m²/cm³ und der Gleichmäßigkeitsparameter n (Steigung im RRSB-Netz) im Mittel 0,8 betrug. Dabei waren 90 Gew.-% der Kohlekörner kleiner als 65 µm, 50 Gew.-% kleiner als 13 µm (in etwa entsprechend der Kurve 1 in Fig. 1). Es wurden fünf verschiedene Versuche durchgeführt, bei denen jeweils die Länge der Reaktionsstrecke zwischen dem Eindüsen der Kohle in das durch den Lichtbogen aufgeheizte Gas und dem Abschrecken durch Verschieben der Aufgabelanze für das Abschreckmedium (Wasserstoff) variiert wurde. Aus den verschiedenen Längen der Reaktionsstrecken wurden die zugehörigen. Verweilzeiten aus der Strömungsgeschwindigkeit unter Berücksichtigung der Gastemperatur berechnet. Für diese fünf Versuche wurden die Acetylenausbeuten bezogen auf das Gewicht der eingesetzten Kohle bestimmt. Die Ergebnisse sind in Tabelle 1 wiedergegeben.

| Länge der Reaktionsstrecke | Berechnete Verweilzeit | Acetylenausbeute |
|---|---|---|
| cm | $10^{-3}$ s | $kgC_2H_2$/kg Kohle |
| 10 | 2,0 | 0,25 |
| 12 | 2,5 | 0,29 |
| 15 | 3,0 | 0,30 |
| 18 | 4,0 | 0,26 |
| 21 | 4,5 | 0,23 |

Die Verweilzeit, bei der die maximale Acetylenausbeute erzielt wurde, lag — wie durch graphische Ermittlung festgestellt wurde — bei dieser Kohleprobe bei etwa $2,8 \times 10^{-3}$ s. Die hierzu gehörige Acetylenausbeute betrug 0,3 kg Acetylen/kg Kohle. Daraus errechnete sich ein spezifischer Energiebedarf (ohne die Verlustenergie im Kühlwasser) von 13,0 kWh/kg Acetylen.

### Beispiel 2

Die Kohle des Beispiels 1 wurde durch Windsichtung in eine feine und eine grobe Kornfraktion zerlegt, wobei 39,7 Gew.-% der eingesetzten Ausgangskohle auf die Feinkornfraktion entfielen. 90 Gew.-% der Feinkornfraktion waren kleiner als 9 μm und 50 Gew.-% kleiner als 4,8 μm; der Gleichmäßigkeitsparameter der RRSB-Teilung betrug 2,0 (etwa Kurve 2 im Bild 1 entsprechend). Diese Fraktion hatte eine Blaine-Oberfläche von 2,3 m²/cm³. Die aus der Windsichtung hervorgegangene grobe Kornfraktion umfaßte 60,3 Gew.-% der eingesetzten Ausgangskohle, wobei 90 Gew.-% aus Körnern kleiner als 50 μm und 50 Gew.-% kleiner als 22 μm (etwa Kurve 3 im Bild 1 entsprechend). Der Gleichmäßigkeitsparameter der Grobkornfraktion betrug 1,4 und die Blaine-Oberfläche wurde zu 0,47 m²/cm³ ermittelt.

Aufgrund des Ergebnisses der in Beispiel 1 beschriebenen Versuche wurde erfindungsgemäß für die feine Kornfraktion eine optimale Verweilzeit berechnet von

$$2,8 \times 10^{-3} \times \sqrt[3]{1,26 : 2,3}\,\text{sec.} = 2,3 \times 10^{-3}\,\text{s}$$

und für die grobe Kornfraktion eine optimale Verweilzeit von

$$2,8 \times 10^{-3} \times \sqrt[3]{1,26 : 0,47}\,\text{sec.} = 3,9 \times 10^{-3}\,\text{s}.$$

Es wurde — entsprechend einer Eichkurve — durch verschiedene Aufgabelanzen für das Abschreckmedium Wasserstoff die Länge der Reaktionsstrecke für die feine Kornfraktion auf etwa 12 cm und für die grobe Kornfraktion auf etwa 18 cm eingestellt und im übrigen die Reaktion, wie weiter oben beschrieben, durchgeführt. Die Ergebnisse sind zusammen mit dem optimalen Ergebnis des Beispiels 1 in Tabelle 2 wiedergegeben.

| Blaine-Oberfläche | Steigung n im RRSB-Netz | Länge der Reaktionsstrecke | Verweilzeit | Acetylenausbeute |
|---|---|---|---|---|
| m²/cm³ | | cm | $10^{-3}$ s | kgC₂H₂/kg Kohle |
| 1,26 | 0,8 | 14 | 2,8 | 0,30 |
| 2,30 | 2,0 | 12 | 2,3 | 0,40 |
| 0,47 | 1,4 | 18 | 3,9 | 0,33 |

Dabei errechnete sich ein spezifischer Energiebedarf (ohne die Verlustenergie im Kühlwasser) von 9,8 kW/kg Acetylen für die Feinkornfraktion und 11,8 kW/kg Acetylen für die Grobkornfraktion.

Berechnet man die gesamte Acetylenausbeute für die Fein- und für die Grobkornfraktion sowie den gesamten spezifischen Energiebedarf unter Berücksichtigung der jeweiligen Gewichtsanteile, so erhält man eine totale Acetylenausbeute von 0,36 kg Acetylen/kg Kohle und ein spezifischen Energiebedarf von 10,9 kW/kg Acetylen. Verglichen mit der Ausbeute und dem spezifischen Energiebedarf der nicht fraktionierten Kohle aus der ersten Versuchsreihe bedeutet das Ergebnis einen erheblichen Vorteil, der sich in einer etwa 19% gestiegenen Acetylenausbeute bei gleichzeitiger Absenkung des spezifischen Energiebedarfes um etwa 16% ausdrückt.

### Patentansprüche

1. Verfahren zur kontinuierlichen Herstellung von Acetylen nach an sich bekannten Methoden in einer Strömung von gemahlener Braun- oder Steinkohle, die sehr schnell auf über 1400° C aufgeheizt und dann mit kaltem Gas, kalten, flüssigen Kohlenwasserstoffen oder Wasser abgeschreckt wird, bei welchem man

a) die Verweilzeit der Kohle in der Strömung zwischen dem Beginn des Aufheizens und dem Abschrecken (Reaktionsstrecke) bei sonst konstanten, vorbestimmten Bedingungen variiert und die sich jeweils ergebende Acetylenausbeute bestimmt,

b) die Verweilzeit ermittelt, bei der die Acetylenausbeute ihren höchsten Wert erreicht (Optimalzeit),

dadurch gekennzeichnet, daß man auf kleiner als 0,5 mm aufgemahlene Kohle verwendet, sie in mindestens zwei Kornfraktionen trennt und aus letzteren jeweils einzeln Acetylen produziert, indem bei sonst gleichen Bedingungen die Verweilzeiten sich jeweils so zu der Optimalzeit verhalten, wie das umgekehrte Verhältnis der etwa dritten Wurzeln der spezifischen äußeren Oberfläche der jeweiligen Kornfraktionen.

2. Verfahren nach Anspruch 1, dadurch gekennzeichnet, daß man die Länge der Reaktionsstrecke variiert.

3. Verfahren nach Anspruch 1, dadurch gekennzeichnet, daß man die Strömungsgeschwindigkeit der Kohle in der Reaktionsstrecke variiert.

4. Verfahren nach einem oder mehreren der Ansprüche 1 bis 3, dadurch gekennzeichnet, daß die spezifische äußere Oberfläche der Kohle (nach der Blaine-Methode bestimmt) zwischen 0,5 und 2,0 m$^2$/cm$^3$ beträgt.

5. Verfahren nach einem oder mehreren der Ansprüche 1 bis 4, dadurch gekennzeichnet, daß bei der Korngrößenverteilung jeder Kohlefraktion die mittlere Steigung der Körnungskennlinie im Rosin-Rammler-Sperling-Bennet-Netz zwischen den Durchgängen von 10% und 80% zwischen 1,2 und 2,5 beträgt.

6. Verfahren nach einem oder mehreren der Ansprüche 1 bis 5, dadurch gekennzeichnet, daß die Kohle einen Gehalt an flüchtigen Bestandteilen, bezogen auf wasser- und aschefreie Basis (i. waf) zwischen 25 und 44%, vorzugsweise zwischen 30 und 40%, und einen Gehalt an organischem Sauerstoff (i. waf) unter 9% aufweist.

7. Verfahren nach einem oder mehreren der Ansprüche 1 bis 6, dadurch gekennzeichnet, daß die Aufheizung in einem Wasserstoff enthaltenden Gasstrom erfolgt.

8. Verfahren nach Anspruch 7, dadurch gekennzeichnet, daß das Wasserstoff enthaltende Gas durch einen Lichtbogen mit einer Energie von 100 bis 500 kJ/mol aufgeheizt wird.

9. Verfahren nach einem oder mehreren der Ansprüche 1 bis 7, dadurch gekennzeichnet, daß die Energie zum Aufheizen durch Verbrennung eines kohlenstoffhaltigen Materials unter weitgehender Vermeidung von CO$_2$ und O$_2$ im Rauchgas bereitgestellt wird.

10. Verfahren nach Anspruch 9, gekennzeichnet durch Rückführung von nach der Reaktionsstrecke anfallenden, kohlenstoffhaltigen Rückständen.

11. Verfahren nach einem oder mehreren der Ansprüche 8 bis 10, dadurch gekennzeichnet, daß das Verhältnis von thermischer Leistung zur Strömung an Kohle 4000 bis 40 000 kJ/kg Kohle beträgt.

12. Verfahren nach Anspruch 1, gekennzeichnet durch die Verwendung eines länglichen Reaktionsraumes (10) für die Durchströmung mit Kohle (8) mit
a) je einer Einspeisestelle für die Kohle (9) und Aufheizenergie (7) an dem einen Endbereich,
b) einer längs des Reaktionsraumes (10) variierbaren Einspeisestelle (13) für ein Abschreckmedium (16) und
c) einem Austritt (15) für Produktgas und Rückstandsmaterial.

13. Verfahren nach Anspruch 12, gekennzeichnet durch eine axial im Reaktionsraum (10) verschiebbare Aufgabelanze (13) für das Abschreckmedium (16).

14. Verfahren nach Anspruch 12, gekennzeichnet durch längs des Reaktionsraumes (10) an seiner Peripherie angeordnete Düsen (9) für das Abschreckmedium (16).

## Claims

1. A process for the continuous preparation of acetylene according to methods know per se in a flow of crushed lignite or coal which is heated very rapidly to over 1400°C and is then quenched with cold gas, cold liquid hydrocarbons, or water, in which
a) the dwell period of the coal in the flow between the beginning of heating and the quenching (reaction period) is varied while the predetermined conditions remain otherwise constant, and the acetylene yield produced in each case is determined,
b) the dwell period in which the acetylene yield reaches its highest value (optimum time) is ascertained, characterised in that coal crushed to less than 0.5 mm is used, it is separated into at least two grain fractions and acetylene is produced separately from the latter in each case, and — under conditions otherwise the same — the dwell periods behave in each case relative to the optimum time like the inverse ratio of the approximately cube roots of the specific outer surface of the respective grain fractions.

2. A process according to claim 1, characterised in that the length of the reaction period is varied.

3. A process according to claim 1, characterised in that the flow velocity of the coal in the reaction period is varied.

4. A process according to one or more of claims 1 to 3, characterised in that the specific outer surface of the coal (determined according to the Blaine method) amounts to between 0.5 and 2.0 m$^2$/cm$^3$.

5. A process according to one or more of claims 1 to 4, characterised in that in the grain size distribution of each coal fraction the mean gradient of the grain size curve in the Rosin-Rammler-Sperling-Bennet net between the passages of 10% and 80% amounts to between 1.2 and 2.5.

6. A process according to one or more of claims 1 to 5, characterised in that the coal has a content of volatile constituents, relative to a water and ash free base, of between 25 and 44%, preferably between 30 and 40%, and a content of organic oxygen (on a water and ash free base) of below 9%.

7. A process according to one or more of claims 1 to 6, characterised in that heating takes, place in a gas stream containing hydrogen.

8. A process according to claim 7, characterised in that the gas containing hydrogen is heated by an electric arc with a power of from 100 to 500 kJ/mol.

9. A process according to one or more of claims 1 to 7, characterised in that the energy for heating is produced by burning a carbonaceous material while largely avoiding $CO_2$ and $O_2$ in the flue gas.

10. A process according to claim 9, characterised by recycling carbonaceous residues occurring after the reaction period.

11. A process according to one or more of claims 8 to 10, characterised in that the ratio of thermal capacity to coal flow amounts to between 4,000 and 40,000 kJ/kg of coal.

12. A process according to claim 1, characterised by the use of an elongate reaction chamber (10), through which the coal (8) flows, with

a) a junction point in each case for the coal (9) and heating energy (7) at one end region,

b) a junction point (13) for a quenching medium (16) which is variable along the reaction chamber (10), and

c) an outlet (15) for product gas and residue material.

13. A process according to claim 12, characterised by a feeding lance (13) which is axially displaceable in the reaction chamber (10), for the quenching medium (16).

14. A process according to claim 12, characterised by nozzles (9) arranged along the reaction chamber (10) on its periphery, for the quenching medium (16).

## Revendications

1. Procédé de production continue d'acétylène suivant des méthodes connues en soi, dans un courant de lignite ou de houille broyée, qui est chauffé très rapidement à plus de 1400°C puis refroidi brusquement par un gaz froid, des hydrocarbures liquides froids ou de l'eau, dans lequel,

a) On fait varier le temps de séjour du charbon dans le courant entre le début du chauffage et le refroidissement brusque (zone de réaction) dans des conditions par ailleurs constantes et prédéterminées et on détermine le rendement en acétylène obtenue chaque fois;

b) On calcule le temps de séjour auquel le rendement en acétylène atteint sa valeur la plus élevée (temps optimal), caractérisé en ce qu'on utilise du charbon broyé plus fin que 0,5 mm on le sépare en au moins deux fractions granulométriques et, à partir de ces fractions, on produit isolément de l'acétylène, cependant que dans des conditions analogues par ailleurs, le rapport de chaque temps de séjour au temps optimal est inversement proportionnel au rapport des racines cubiques des surfaces extérieures spécifiques des fractions granulométriques correspondantes.

2. Procédé suivant la revendication 1, caractérisé en ce qu'on fait varier la longueur de la zone de réaction.

3. Procédé suivant la revendication 1, caractérisé en ce qu'on fait varier la vitesse d'écoulement du charbon dans la zone de réaction.

4. Procédé suivant l'une ou plusieurs des revendications 1 à 3, caractérisé en ce que la surface extérieure spécifique du charbon (déterminée par la méthode Blaine) est de 0,5 à 2,0 m²/cm³.

5. Procédé suivant l'une ou plusieurs des revendications 1 à 4, caractérisé en ce que, dans la distribution granulométrique de chaque fraction du charbon, la pente moyenne de la ligne caractéristique de granulométrie dans le réseau Rosin-Rammler-Sperling-Bennet es comprise entre 1,2 et 2,5 entre les fractions passantes de 10% et de 80%.

6. Procédé suivant l'une ou plusieurs des revendications 1 à 5, caractérisé en ce que le charbon présente une teneur en constituants volatils, rapportée à une base sans eau ni cendres (senc) entre 25 et 44%, de préférence entre 30 et 40% et une teneur en oxygène organique (senc) inférieure à 9%.

7. Procédé suivant l'une ou plusieurs des revendications 1 à 6, caractérisé en ce que le chauffage se produit dans un courant gazeux contenant de l'hydrogène.

8. Procédé suivant la revendication 7, caractérisé en ce que le gaz contenant de l'hydrogène est chauffé par un arc électrique ayant une énergie de 100 à 500 kJ/mole.

9. Procédé suivant une ou plusieurs des revendications 1 à 7, caractérisé en ce que l'énergie pour le chauffage est produite par combustion d'une matière carbonée en évitant largement la présence de $CO_2$ et de $C_2$ dans les gaz de fumée.

10. Procédé suivant la revendication 9, caractérisé par le recyclage des résidus carbonés qui se présentent en aval de la zone de réaction.

11. Procédé suivant une ou plusieurs de revendications 8 à 10, caractérisé en ce que le rapport de la puissance thermique au courant de charbon est de 4000 à 40 000 kJ/kg de charbon.

12. Procédé suivant la revendication 1, caractérisé par l'utilisation d'une chambre de réaction allongée (10) destinée à être parcourue par du charbon (8) comprenant:

a)   un point d'injection du charbon (9) et de l'énergie de chauffage (7) dans une des régions terminales,

b)   un point d'injection (13) pour un milieu de refroidissement brusque (16) qu'on peut faire varier le long de la chambre de réaction (10) et,

c)   une sortie (15) pour le gaz produit et la matière résiduelle.

13. Procédé suivant la revendication 12, caractérisé par une lance débitrice (15) destinée à l'injection du milieu de refroidissement (13), qu'on peut déplacer axialement en translation dans la chambre de réaction (10).

14. Procédé suivant la revendication 12, caractérisé par des buses (9) pour l'injection du milieu de refroidissement brusque (16) qui sont disposées le long de la chambre de réaction (10) sur sa périphérie.

FIG.1

11

FIG. 2